## Europäisches Patentamt
## European Patent Office
## Office européen des brevets

(19)

(11) Veröffentlichungsnummer : **0 072 823**
**B1**

(12)

# EUROPÄISCHE PATENTSCHRIFT

(45) Veröffentlichungstag der Patentschrift :
17.07.85

(21) Anmeldenummer : 82900544.6

(22) Anmeldetag : 13.02.82

(86) Internationale Anmeldenummer :
PCT/EP 82/00027

(87) Internationale Veröffentlichungsnummer :
WO/8202822 (02.09.82 Gazette 82/21)

(51) Int. Cl.⁴ : **A 61 B   5/02**, G 01 D 15/10

(54) VORRICHTUNG ZUR MESSUNG UND REGISTRIERUNG DES BLUTDRUCKS.

(30) Priorität : 19.02.81 DE 3106026

(43) Veröffentlichungstag der Anmeldung :
02.03.83 Patentblatt 83/09

(45) Bekanntmachung des Hinweises auf die Patenterteilung : 17.07.85 Patentblatt 85/29

(84) Benannte Vertragsstaaten :
AT BE CH DE FR GB LI

(56) Entgegenhaltungen :
DE-A- 2 454 195
DE-A- 2 525 743
DE-A- 2 754 333
FR-A-   732 527
FR-A- 1 423 938
FR-A- 2 239 973
FR-A- 2 374 011
US-A- 3 146 777
US-A- 3 894 533
US-A- 4 145 698
US-A- 4 276 886

(73) Patentinhaber : HESTIA PHARMA GMBH
Oppauer Strasse 130
D-6800 Mannheim 31 (DE)

(72) Erfinder : REBBE, Klaus
Bodelschwinghweg 23
D-6800 Mannheim 31 (DE)
Erfinder : SCHMIDT, Werner
Meerfeldstrasse 46
D-6800 Mannheim 1 (DE)
Erfinder : WELLMANN, Klaus
Cornelius-Heyl-Strasse 5
D-6520 Worms 1 (DE)

(74) Vertreter : Weber, Manfred, Dr. et al
Boehringer Mannheim GmbH Patentabteilung Sandhofer Strasse 116
D-6800 Mannheim 31 (DE)

## Beschreibung

Die vorliegende Erfindung betrifft eine Vorrichtung zur Messung und Registrierung des Blutdrucks, umfassend eine aufblasbare Druckmanschette zum Stauen des Blutes, eine Empfangseinrichtung zum Empfangen der Korotkoff-Töne und Umwandeln derselben in elektrische Signale, eine Signalverarbeitungseinrichtung zum Aufbereiten der elektrischen Signale, eine Druckmeßeinrichtung zum Messen des Druckes in der Manschette und eine Registriereinrichtung zur dauerhaften Aufzeichnung der Blutdruckwerte durch Markieren von mindestens zwei Druckwerten pro Messung mit Hilfe einer Markierungseinrichtung auf einem Registrierblatt mit einer druckgraduierten Skala, wobei die beiden Druckwerte näherungsweise dem ersten und letzten empfangenen Korotkoff-Ton, also dem systolischen und diastolischen Blutdruck entsprechen.

Schon seit langem besteht ein Bedürfnis, den Blutdruck von Patienten nicht nur zu messen, sondern dauerhaft zu registrieren. Zum einen könnte man die in irgendeiner Form aufgezeichneten Blutdruckmeßwerte in Ruhe und sorgfältig ablesen und damit eine verbesserte Meßgenauigkeit erzielen. Zum anderen aber ließen sich solche registrierten Blutdruckmeßwerte zur späteren Verwendung aufbewahren. Insbesondere bei der Selbstmessung des Blutdruckes durch den Patienten ist dies von erheblicher Bedeutung, da er die registrierten Ergebnisse seiner Blutdruckmessung dem Arzt vorlegen könnte. Eine Registrierung sollte möglichst so weit automatisiert erfolgen, daß der Patient keinen Einfluß auf die registrierten Meßergebnisse hat.

In der normalen Umgebung des Patienten gemessene und ohne dessen Einfluß registrierte Blutdruckmeßgeräte haben auch aus medizinischer Sicht erhebliche Bedeutung. Es ist nämlich festgestellt worden, daß Blutdruckmeßergebnisse, die in der Praxis des Arztes gewonnen werden, häufig dadurch verfälscht werden, daß der Patient sich in einem nicht seinen normalen Lebensumständen entsprechenden Zustand befindet. Die Selbstmessung zu Hause andererseits leidet bei Fehlen einer gleichzeitigen Registrierung dadurch, daß der Patient häufig, im allgemeinen unbewußt, Blutdruckmeßwerte nicht ganz korrekt aufschreibt, oder in vielen Fällen das Aufschreiben ganz vergißt.

Wegen dieses dringenden Bedürfnisses hat es bereits eine Reihe von Versuchen gegeben, eine dauerhafte Registrierung von Blutdruckmeßwerten durch Einrichtungen der eingangs genannten Art zu erreichen. Aus der DE-OS 23 40 813 ist ein Blutdruckregistriergerät mit den eingangs aufgeführten Merkmalen bekannt. Bei dieser Einrichtung wird der Staudruck der Blutdruckmanschette mit Hilfe einer Druckdose und eines dieser nachgeschalteten Drehmomentgebers in die Drehbewegung eines Zeigers umgewandelt. Am Ende des Zeigers befindet sich ein elektrisch beheizbarer Schreibstift. Die gesamte Einheit aus Druckdose, Drehmomentgeber und Zeiger ist an einem Rahmen befestigt, der in Abhängigkeit von den Korotkoff-Tönen mit Hilfe zweier Elektromagnete bewegt werden kann.

Zur Messung wird die um den Arm gelegte Blutdruckmanschette wie üblich auf einen Druck oberhalb des systolischen Blutdrucks aufgepumpt und langsam abgelassen. Dabei bewegt sich der Zeiger in Abhängigkeit vom Blutdruck kreisförmig über einem Aufzeichnungsträger. Wenn nun bei Erreichen des systolischen Blutdrucks Korotkoff-Töne auftreten, werden sie mit Hilfe eines Mikrofons und einer elektrischen Schaltung in Signale für die Elektromagneten umgesetzt, dergestalt, daß diese den Träger der Aufzeichnungseinrichtung auf den Aufzeichnungsträger zu bewegen. Dabei berührt der elektrisch beheizbare Schreibstift den Aufzeichnungsträger und hinterläßt eine Markierung auf demselben. In entsprechender Weise führt jeder Korotkoff-Ton zu einer Markierung, so lange bis die Korotkoff-Töne verschwinden, was dem diastolischen Blutdruck entspricht. Jetzt werden die Magnete nicht mehr erregt und demzufolge auch keine Markierungen mehr erzeugt. Der Aufzeichnungsträger auf dem die Markierungen erzeugt werden, ist mit einer entsprechenden Druckgraduierung versehen, so daß die Reihe der Markierungen zum Ablesen des systolischen und diastolischen Blutdrucks verwendet werden kann.

In der Praxis hat sich gezeigt, daß die vorbeschriebene bekannte Einrichtung erhebliche Nachteile aufweist. Insbesondere muß die Führung des Tragrahmens für Druckdose und Drehmomentgeber präzise gefertigt sein, um eine einwandfreie Markierung zu erreichen und ein Verkanten zu vermeiden. Der Tragrahmen hat eine große träge Masse. Demzufolge müssen die Magneten sehr kräftig ausgeführt sein, woraus sich ein zu hoher Energieverbrauch ergibt. Dies führte zur Entwicklung der in der DE-OS 26 57 612 beschriebenen Vorrichtung, die der vorbeschriebenen sehr ähnlich ist. Auch hier wird der Luftdruck über eine Druckmeßdose und einen Drehmomentgeber in eine Drehbewegung eines Markierungsstiftes umgesetzt. Die Bewegung des Markierungsstiftes auf den Aufzeichnungsträger zu bei Auftreten eines Korotkoff-Tones wird in diesem Fall aber dadurch erreicht, daß der Zeiger an einem elastisch nachgiebigen Lager befestigt ist und magnetisch von einem unter dem Aufzeichnungsträger angeordneten Magneten angezogen werden kann. Bei dieser Konstruktion ist die träge Masse, die bewegt werden muß, erheblich geringer, jedoch ist auch dieses bekannte Gerät sehr aufwendig herzustellen.

Außerdem ermöglicht dieses bekannte Blutdruck-Registriergerät nur die Aufzeichnung einer einzigen Blutdruckmessung, während, wie oben beschrieben, vielfach gerade die Aufzeichnung mehrerer Blutdruckmessungen auf einem Aufzeichnungsträger, dergestalt, daß sich eine Verlaufsanzeige ergibt, medizinisch sehr wünschenswert ist.

Aufgabe der vorliegenden Erfindung ist es daher, eine einfache, zuverlässige Registrierung mehrerer Blutdruckmeßergebnisse für Patient und Arzt zu ermöglichen.

Diese Aufgabe wird erfindungsgemäß bei einer Einrichtung der eingangs erwähnten Art dadurch gelöst, daß das Registrierblatt Bereiche für eine Mehrzahl von zu verschiedenen Zeitpunkten gewonnenen Meßergebnissen aufweist, die Markierungseinrichtung einen relativ zum Registrierblatt, insbesondere manuell, beweglichen und in verschiedene Positionen einstellbaren Schlitten aufweist, wobei jede Position einem verschiedenen Meßzeitpunkt entspricht, an dem Schlitten eine Mehrzahl insbesondere punktförmige Markierungen erzeugende Druckelemente in einer relativ zum Schlitten festen Position angebracht sind, wobei jedes Element einem bestimmten Druckbereich der Druckmeßeinrichtung entspricht, die Signalverarbeitungseinrichtung digitalisierende Elemente zur Erzeugung definierter Impulse aus den Korotkoff-Geräuschen aufweist und eine Ansteuereinrichtung vorgesehen ist, durch die die Druckelemente angesteuert werden, wenn in dem dem jeweiligen Element entsprechenden Druckbereich ein mindestens dem ersten und dem letzten empfangenen Korotkoff-Ton entsprechender definierter Impuls von der Signalverarbeitungseinrichtung empfangen wird.

Die eigentliche Blutdruckmeßeinrichtung ist in bekannter Weise aufgebaut. Bevorzugt wird für die Erfindung ein digitales elektronisches Blutdruckmeßgerät verwendet, dessen Blutdruckmanschette mit einem Mikrofon als Schallaufnehmer versehen ist. Der Blutdruck wird über einen Druckwandler gemessen, der dem in der Manschette herrschenden Staudruck entsprechende elektrische Signale erzeugt. Die den Korotkoff-Tönen entsprechenden elektrischen Signale des Mikrofons werden in einer elektronischen Schaltung aufbereitet. Dabei werden vielfach Filter und andere elektronische Schaltelemente benutzt, um die Korotkoff-Geräusche von Störgeräuschen zu unterscheiden. Bei den modernsten Geräten dieser Art werden die Druckwerte auf einer digitalen Anzeige zur Anzeige gebracht. Zu diesem Zweck werden die Korotkoff-Töne und die Signale des Druckwandlers elektronisch korreliert. Die Druckwerte, bei denen der erste Korotkoff-Ton auftaucht und bei denen die Korotkoff-Geräusche verschwinden, werden in einem elektronischen Speicher abgespeichert und angezeigt. Einzelheiten der Ausführung des Blutdruckmeßgerätes sind jedoch für die vorliegende Erfindung nicht entscheidend. Diese richtet sich vielmehr auf die Registrierung der Blutdruckmeßwerte und die Konstruktion des zugehörigen Blutdruckmeßgerätes ist nur insoweit bedeutungsvoll, als sie in einer auf die erfindungsgemäße Blutdruckregistriereinrichtung angepaßten Weise ausgeführt sein sollte. In die Erfindung eingeschlossen sind demzufolge sowohl Geräte, die die Blutdruckmessung und -registrierung in einer Einheit verwirklichen, als auch solche Geräte, die als Zusatz zu bereits vorhandenen Blutdruckmeßgeräten eine erfindungsgemäße Registrierung ermöglichen.

Die zeitlichen Bereiche auf dem Registrierblatt müssen nicht notwendigerweise, beispielsweise durch einen entsprechenden Aufdruck, ohne weiteres erkennbar sein. Gemeint ist damit vielmehr, daß die Registrierung der zu verschiedenen Zeitpunkten gewonnenen Blutdruckmeßergebnisse räumlich getrennt auf dem Registrierblatt erfolgt, wobei der Schlitten in entsprechender Weise zwischen zwei aufeinanderfolgenden Registriervorgängen relativ zu dem Registrierblatt bevorzugt manuell bewegt wird. Unter dem Begriff «Schlitten» ist jeder das Registrierblatt überspannende Träger zu verstehen, der geeignet ist, die Druckelemente über dem Registrierblatt in der für den Druckvorgang nötigen Position zu halten. Die relative Bewegung von Schlitten und Registrierblatt geschieht bevorzugt dadurch, daß der Schlitten in geeigneten Führungen, beispielsweise einer Schwalbenschwanzführung oder einer formschlüssigen Flachführung, in sich parallel über dem Registrierblatt beweglich ist. Sie kann aber auch durch ein, gegebenenfalls mit einer geeigneten Halterung, bewegliches Registrierblatt erreicht werden, welches relativ zu einer festen, die Druckelemente tragenden Trägerbrücke, verschoben werden kann.

Die erfindungsgemäße Vorrichtung kommt mit einem Minimum an beweglichen Teilen aus. Dadurch kann der Energieverbrauch so weit gesenkt werden, daß gegebenenfalls eine Batterie zur Energieversorgung ausreicht. Als Druckelemente kommen beispielsweise elektromagnetische Einrichtungen mit einem kleinen Bewegungsweg in Frage. Die Markierungen selbst werden beispielsweise durch konventionelle Schreiberstifte erzeugt.

Gemäß eines besonders bevorzugten Ausführungsform sind die Druckelemente bewegungslos ausgeführt. Beispielsweise kann hierfür das elektrosensitive Druckprinzip verwendet werden, bei dem auf einem Spezialpapier gedruckt wird, dessen Zelluloseträger mit einer durchgehenden, gleichmäßigen Aluminiumschicht bedampft ist. Zwischen Aluminiummetallisierung und Zellulose befindet sich eine schwarze Kontrastschicht, die durch punktförmiges Abbrennen der dünnen Aluminiumschicht sichtbar wird. Bei einer derartigen Ausführung enthält die erfindungsgemäße Registriereinrichtung praktisch keine beweglichen Teile mehr, so daß der Energieverbrauch besonders gering und die Zuverlässigkeit besonders hoch ist. Derartige Druckeinrichtungen sind auch zu verhältnismäßig günstigen Kosten herzustellen. Wegen des geringen Energieverbrauches ist eine Ausführung als Batteriegerät möglich. Besonders in diesem Zusammenhang ist eine Ausführungsform zu bevorzugen, bei der die Relativbewegung von Registrierblatt und Schlitten manuell erfolgt, weil eine derartige Lösung besonders zuverlässig und energiesparend ist. Insbesondere für stationäre Geräte kommen aber auch Ausführungsformen mit automatischer, insbesondere elektromotorischer Verstellung des Schlittens oder des Registrierblattes in Betracht.

Bevorzugt ist die bei bewegungslosen Druckelementen notwendige Andruckeinrichtung als im Schlitten untergebrachte, das Substrat der Druckelemente belastende Federeinrichtung ausgeführt. Alternativ dazu kann die Andruckrichtung auch unter dem Registrierblatt vorgesehen sein und dieses gegen die Druckelemente pressen. Die erstgenannte Ausführungsform hat den Vorteil, daß der Schlitten zum Einlegen des Registrierblattes besonders leicht aufklappbar ausgeführt werden kann.

Gemäß einer weiteren bevorzugten Ausführungsform werden thermisch druckende Druckelemente verwendet. Derartige Druckeinrichtungen haben sich insbesondere für kleine elektronische Rechner bewährt, da sie kostengünstig und zuverlässig arbeiten und ein gut lesbares Schriftbild erzeugen. Außerdem ist das Druckpapier vergleichsweise preiswert.

Die Ansteuereinrichtung für die Druckelemente kann derart ausgeführt sein, daß sämtliche Druckelemente, die im Bereich zwischen systolischem und diastolischem Blutdruck bei einer bestimmten Messung liegen, auf dem Registrierblatt eine Markierung erzeugen. Gemäß einer bevorzugten Ausführungsform kann die Ansteuereinrichtung eine Speicherschaltung einschließen, die es erlaubt, den Druckbereich, der dem systolischen bzw. diastolischen Blutdruck entspricht, abzuspeichern und dann nur die Druckelemente anzusteuern, die diesem Druckbereich entsprechen. Eine derartige Einrichtung kann bezüglich des grafischen Bildes und damit der Ablesbarkeit der Registrierung Vorteile aufweisen.

Es sei in diesem Zusammenhang darauf hingewiesen, daß die Qualität der Registrierung selbstverständlich mit der Anzahl der Druckelemente an dem Schlitten steigt. Die Auflösung der Registrierung entspricht dem Quotienten aus dem Druckbebereich, der zur Anzeige kommt, und der Anzahl der Druckelemente. Insbesondere bei Verwendung moderner bewegungsloser Druckelemente ist ohne weiteres eine sehr hohe Elementzahl zu realisieren, die durch eine entsprechende Matrix in dem Fachmann ohne weiteres zugänglicher Art und Weise über eine verhältnismäßig kleine Zahl von Steuerleitungen angesteuert werden können. In der Praxis können ohne weiteres mehrere hundert Druckelemente zur Anwendung kommen, so daß der Druckbereich, der einem Druckelement entspricht, bei einem Gesamtmeßbereich von 300 mm Hg etwa 1 mm Hg entspricht. Eine derartige Auflösung ist bereits besser als die mit der Methode nach Riva-Rocci-Korotkoff im allgemeinen zu erreichende Meßgenauigkeit, so daß es sinnvoll sein kann, die Zahl der Druckelemente bewußt kleiner zu halten, um ein klares Registrierbild zu erreichen. Die erfindungsgemäße Registrierung ist zwar gemäß dem heutigen Stand der Erkenntnisse auf die Blutdruckmessung nach Riva-Rocci-Korotkoff ausgerichtet, jedoch prinzipiell auch für andere Methoden der Blutdruckmessung mit geringen Abwandlungen geeignet.

Gemäß weiteren bevorzugten Ausführungsformen der Erfindung ist das Registrierblatt im wesentlichen rechteckig ausgeführt, wobei die Bereiche für die zu verschiedenen Zeitpunkten gewonnenen Meßergebnisse spaltenförmig, gerade und parallel verlaufen und eine für alle Bereiche gleiche Druckskalierung vorgesehen ist, so daß die Markierungen ein quasikontinuierliches Bild des Verlaufs des Blutdruckes gegen die Zeit ergeben. Bevorzugt ist das Registrierblatt für zwei tägliche Blutdruckmessungen aufgeteilt, wobei der Gesamtzeitraum bis zu einem Monat betragen kann. Der Druckbereich liegt bei bevorzugt 0 bis 300 mm Hg, eventuell sind auf dem Registrierblatt Felder für weitere Angaben vorgesehen, beispielsweise persönliche Angaben über den Benutzer. Die Druckelemente auf dem Schlitten sind bevorzugt in gleichmäßigen Abständen angeordnet und entsprechen äquidistanten Druckbereichen, so daß sich eine lineare Blutdruckanzeige ergibt. Eine derartige lineare Anzeige ist bei der erfindungsgemäßen Einrichtung durch die erwähnten Maßnahmen leicht zu realisieren, während bei den bekannten Einrichtungen die Anzeige und Registrierung häufig nicht linear und damit weniger leicht ablesbar war.

Zusätzlich zur Registriereinrichtung kann eine digitale Anzeige der Blutdruckwerte vorhanden sein. Diese besonders leicht ablesbare Anzeige wird dann zur Darstellung des jeweils gemessenen aktuellen Blutdruckwertes benutzt, während die Registrierung der Verlaufskontrolle dient. Es kann aber auch die Registriereinrichtung gleichzeitig als einzige Blutdruckanzeige verwendet werden, was zu einem besonders kostengünstigen Blutdruckmeß- und Registriergerät führt.

Bevorzugt schließt die Elektronik der erfindungsgemäßen Gerätes, insbesondere die Ansteuereinrichtung, einen Mikroprozessor ein, der gleichzeitig zur Fehlererkennung der von dem Manschettenmikrofon kommenden elektrischen Signale verwendet wird. Dies führt zu einem besonders einfachen und gleichzeitig wirkungsvollen Aufbau der Elektronik des Gerätes.

Die Erfindung wird im folgenden anhand eines in den Figuren dargestellten Ausführungsbeispiels näher erläutert. Es zeigen :

Figur 1 eine schematische perspektivische Ansicht eines Blutdruckmeßgerätes, welches sich mit einer erfindungsgemäßen Registriereinrichtung kombinieren läßt,

Figur 2 eine erfindungsgemäße Registriereinrichtung in schematischer perspektivischer Ansicht,

Figur 3 einen Querschnitt entlang der Linie II-II in Fig. 2,

Figur 4 ein Blockschaltbild zur Erläuterung der Funktion der erfindungsgemäßen Vorrichtung und

Figur 5 ein Blockschaltbild einer anderen Ausführungsform der erfindungsgemäßen Vorrichtung unter Verwendung eines Mikroprozessors.

Figur 6 Zeigt eine alternative Ausführungsform einer erfindungsgemäßen Einrichtung.

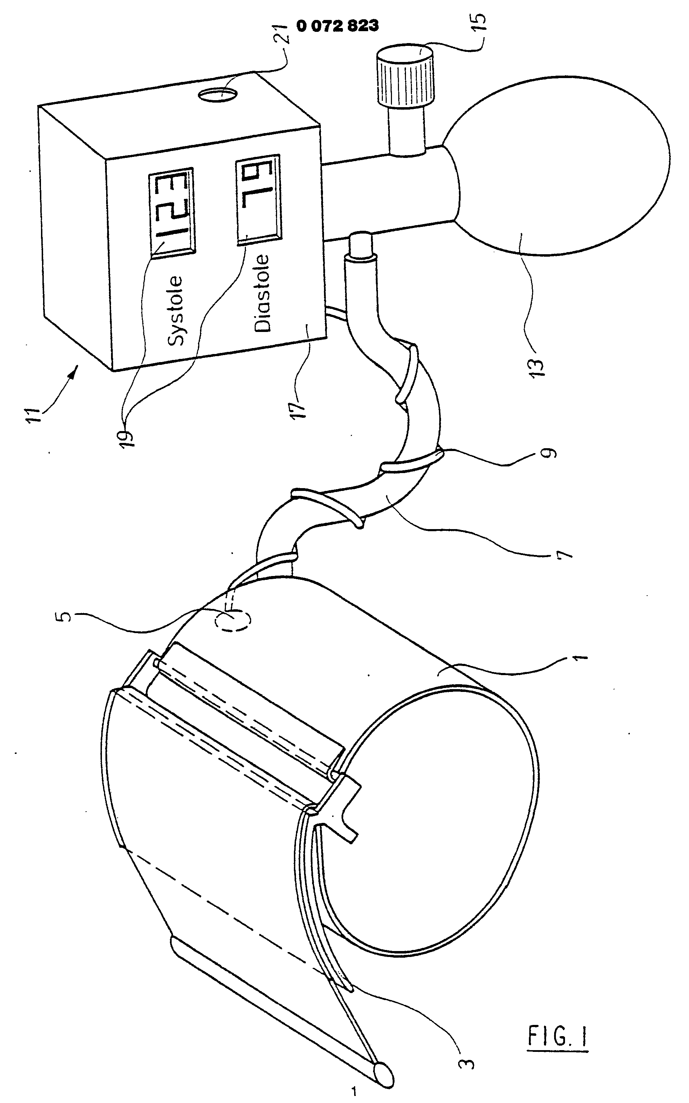

Fig. 1 zeigt die wesentlichen Teile eines digitalen elektronischen Blutdruckmeßgerätes, das zur Verwendung mit einer erfindungsgemäßen Registriervorrichtung vorbereitet ist. Man erkennt die Druckmanschette 1, die sich mit Hilfe eines Bügels 3 und eines nicht dargestellten Klettenbelages bevorzugt am Arm des zu untersuchenden Patienten befestigen läßt. An der Manschette 1 ist ein Mikrofon 5 so befestigt, daß es in engem Kontakt über der Arteria brachialis des Patienten liegt. Von der Manschette weg führt ein Druckschlauch 7 mit einem Mikrofonkabel 9 zu einer Anzeige- und Pumpeinheit, die in ihrer Gesamtheit mit dem Bezugszeichen 11 versehen ist.

Die Anzeige- und Pumpeinheit 11 besteht im wesentlichen aus einem Pumpball 13 zum Aufpumpen der Manschette, einer Druckablaßschraube 15 zum Ablassen des Luftdruckes und einem Anzeigekopf 17, der das Fenster 19 für die digitale Anzeige der Blutdruckwerte aufweist und als Gehäuse für die elektronischen Bauteile des Gerätes dient. Bei besonders modernen Geräten ist statt der Druckablaßschraube 15 eine Tastenbetätigung vorgesehen, die ein geregeltes Ablassen des Luftdruckes mit gleichbleibender Geschwindigkeit ermöglicht. Am Anzeigekopf 17 befindet sich eine Anschlußbuchse 21 zum Anschluß der erfindungsgemäßen Registriervorrichtung.

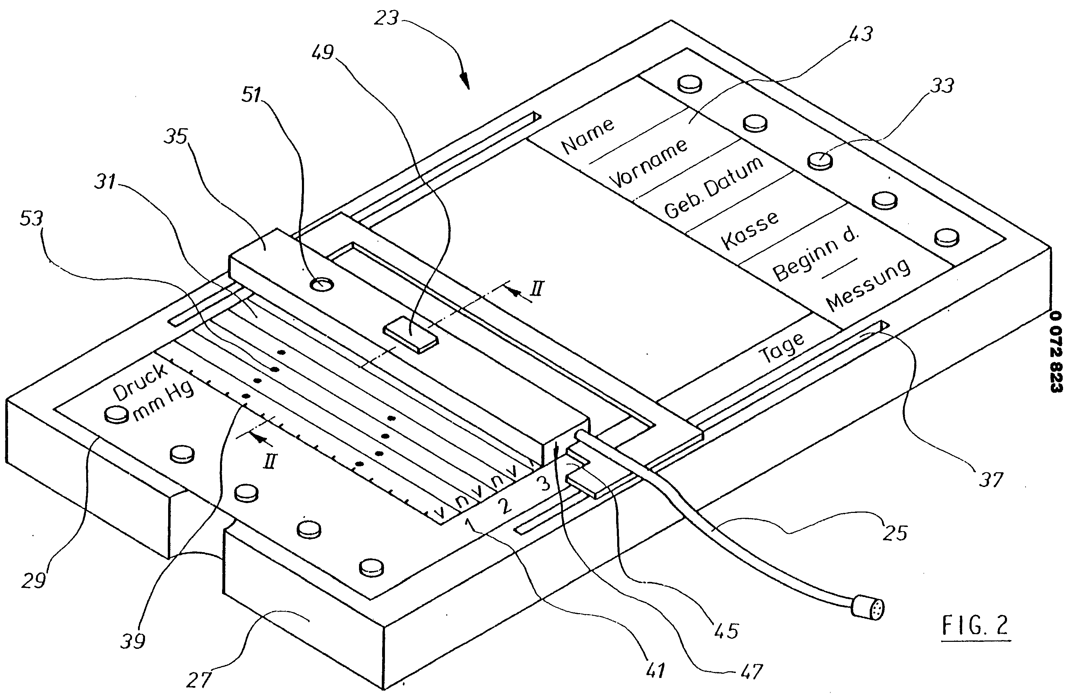

Fig. 2 zeigt die in ihrer Gesamtheit mit dem Bezugszeichen 23 versehene Registriervorrichtung. Sie wird über ein Kabel 25 an die Anschlußbuchse 21 angeschlossen. Die Registriervorrichtung 23 hat ein vorzugsweise aus Kunststoff gefertigtes Gehäuse 27. Das Gehäuse hat eine der Form der vorzugsweise als Registrierblatt verwendeten Karten angepaßte Ausnehmung 29, in die das Registrierblatt 31 eingelegt werden kann. Das Registrierblatt 31 hat kreisrunde Löcher, in welche Haltezapfen 33 eingreifen können, die am Gehäuse 27 vorgesehen sind, um eine genaue Justierung des Registrierblattes 31 zu erreichen.

Über dem Registrierblatt 31 ist ein Schlitten 35 angeordnet, welcher in Führungen parallel zur einen Kante des rechteckig ausgeführten Registrierblattes 31 bewegbar ist. Von den Führungen sind in der Zeichnung lediglich Führungsschlitze 37 zu erkennen. Bei hohen Ansprüchen an die Präzision ist die eigentliche Führung in dem Gehäuse unter den Schlitzen 37 untergebracht und, beispielsweise als Schwalbenschwanzführung, aus Metall gefertigt.

Das Registrierblatt 31 hat eine vertikale Druckgraduierung 39 in mm Hg und eine horizontale Zeitgraduierung 41, welche bevorzugt in 30 Tage eingeteilt ist, wobei jeder Tag zweifach unterteilt ist, um eine morgendliche und abendliche Blutdruckmessung zu ermöglichen. Weiterhin sind auf dem Registrierblatt 31 zusätzliche Informationsfelder 43 vorgesehen, auf denen beispielsweise persönliche Daten des Benutzers, wie sein Name, sein Geburtsdatum, seine Krankenkasse und Angaben über den Meßzeitraum eingetragen werden können.

Der Schlitten 35 läßt sich vom Benutzer über den dem jeweiligen Meßzeitpunkt zugeordneten Bereich des Registrierblattes 31 einstellen. Zu diesem Zweck hat er ein Sichtfenster 45 und einen Justierpfeil 47. Zusätzlich rastet der Schlitten 35 über jedem Bereich leicht ein, um das Einstellen der richtigen Position für den Benutzer zu erleichtern.

Der Schalter 49 zum Einschalten des Gerätes ist ebenfalls am Schlitten 35 vorgesehen. Er erfüllt eine doppelte Funktion. Neben dem elektrischen Einschalten des Gerätes dient er über eine nicht näher dargestellte Rastmechanik dazu, den Andruck der zum Drucken verwendeten Thermoleiste zu erhöhen, wie später beschrieben wird. Zur Anzeige des Betriebszustandes ist eine Anzeigelampe 51 vorgesehen.

Die in dem Schlitten in einer relativ zu diesem festen Position untergebrachten Druckelemente erzeugen auf dem Registrierblatt 31 Markierungspunkte 53, wobei in der Figur eine Ausführungsform dargestellt ist, bei der die Markierungspunkte 53 nur jeweils die dem systolischen und diastolischen Blutdruck entsprechenden Druckwerte markieren. Gemäß einer anderen Ausführungsform der Erfindung wird zwischen systolischem und diastolischem Blutdruckwert jedes Markierungselement angeregt, so daß eine Reihe von Markierungspunkten in vertikaler Richtung erzeugt wird, wobei das obere Ende jeder vertikalen Reihe von Markierungspunkten den zystolischen, das untere Ende den diastolischen Blutdruck anzeigt. Da die die Markierungspunkte erzeugenden Druckelemente erfindungsgemäß in einer relativ zum Schlitten 35 festen Position angeordnet sind, liegen in horizontaler Richtung alle von einem bestimmten Druckelement erzeugten Markierungspunkte auf gleicher Höhe.

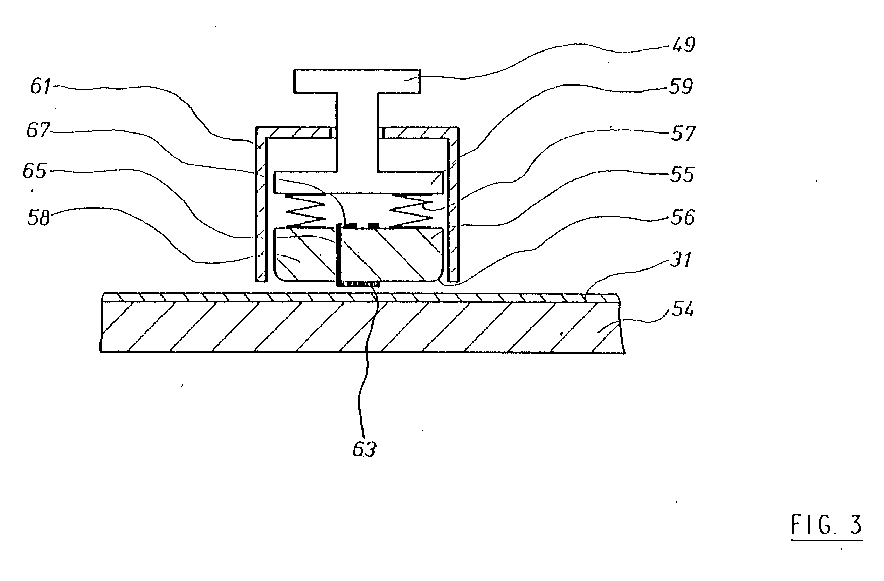

In Fig. 3 ist schematisch ein Querschnitt entlang der Linie II-II in Fig. 1 dargestellt, anhand dessen die prinzipiellen Bauteile des Schlittens 35 näher erläutert werden sollen. Man erkennt die Auflage 54 des Registrierblattes 31. Auf diesem liegt eine Thermoleiste 55 auf, welche mit Hilfe von Federn 57 und einer mit der Taste des Schalters 49 verbundenen Druckplatte 59 gegen das Registrierblatt 31 gedrückt wird. Die Druckplatte 59 stützt sich ihrerseits über eine der Übersichtlichkeit halber nicht dargestellte Rastmechanik des Schalters 49 gegen das Gehäuse 61 des Schlittens 35 ab.

Die Druckplatte 59 und die Thermoleiste 55 sind in dem Gehäuse 61 mit Hilfe von nicht näher dargestellten Führungen in sich parallel dergestalt geführt, daß sie in vertikaler Richtung leicht und ohne zu verkanten beweglich sind. Die Thermoleiste 55 besteht aus einem isolierenden Substrat 58, an dessen Unterseite sich als eigentliche Druckelemente Heizwiderstände 63 befinden. Die Heizwiderstände erhalten ihren Strom, wenn die entsprechenden Elemente angesteuert werden, über Leitungen 65, die in dem Substrat 58 eingebettet sind. An der Oberkante des Substrates können sich weitere Leiterbahnen 67 befinden, die zur Ansteuerungsmatrix der als Druckelemente dienenden Heizwiderstände 63 gehören.

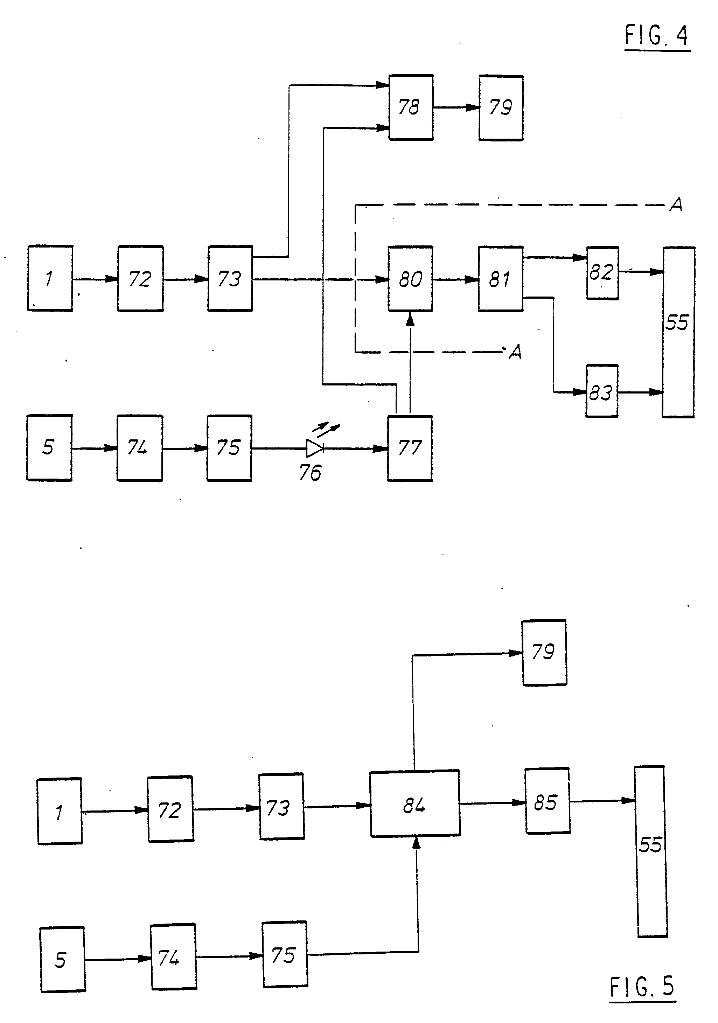

Fig. 4 zeigt als Blockschaltbild die funktionswichtigen Elemente der erfindungsgemäßen Vorrichtung, wobei die einzelnen Blöcke teilweise rein mechanische, teilweise mechanisch-elektrische und teilweise rein elektronische Bauteile bezeichnen. Die Blöcke sollen eine funktionale Zusammenfassung von Bauelementen verdeutlichen, ohne daß dem eine entsprechende räumliche Anordnung der Bauelemente in der konkreten Realisierung entsprechen muß. Eine gestrichelte Linie A-A trennt die Blöcke, die bei der zuvor dargestellten bevorzugten Ausführungsform in den in Fig. 1 dargestellten Bauteilen untergebracht sind, von denen, die sich in der Registriervorrichtung nach Fig. 2 befinden. Auch diese Aufteilung ist zwar besonders zweckmäßig, jedoch keinesfalls beschränkend zu verstehen.

Von der Druckmanschette 1 wird der jeweils herrschende Luftdruck an einen Druckwandler 72 geleitet, der das Herzstück der Druckmeßeinrichtung bildet. Der Druckwandler 72 wandelt den Luftdruck in ein analoges elektrisches Signal, beispielsweise eine Spannung, um, die an einem Analog/Digital-Wandler 73 anliegt, an dessen Ausgang in digitaler Form der jeweils in der Druckmanschette 1 herrschende Druck zur Verfügung steht.

Ein zweiter Signalweg dient der Verarbeitung der Korotkoff-Töne. Das Mikrofon 5 liefert sein Signal an eine Schaltung zur Signalverstärkung und Filterung 74, dessen Ausgang einem Schmitt-Trigger 75 zugeleitet wird. Die Schaltung 74 und der Schmitt-Trigger 75 bilden die Signalverarbeitungseinrichtung, die in vielerlei Weise ausgeführt sein kann, und an deren Ausgang bei jedem Korotkoff-Ton ein definiertes elektrisches Signal auftritt. Dieses kann durch eine Leuchtdiode 76 unmittelbar angezeigt werden und wird an eine Anzeige- und Registrieransteuerung 77 angelegt.

Die Anzeige- und Registrieransteuerung 77 dient einerseits zur Ansteuerung einer digitalen Blutdruckanzeige, die aus einer digitalen Signalverarbeitung 78 und dem eigentlichen Display 79 besteht. Andererseits dient sie auch zur Ansteuerung der Registriereinrichtung, die hintereinander einen Zwischenspeicher 80, einen PROM-Umkodierer 81, Zeilentreiber 82 und Spalten̈̈̈ber 83 und die Printleiste 55 einschließt. Im ̈̈̈rieb liefert der A/D-Wandler 73 sein Ausgangssignal beispielsweise über eine 10 bit-Leitung, die eine Digitalisierung in 1 024 Schritte ermöglicht, einerseits an die digitale Signalverarbeitung 78 und andererseits an den Zwischenspeicher 80. Es liegt also sowohl am Eingang der digitalen Anzeige 78, 79 als auch am Eingang der Registrierschaltung 80 bis 83, 55 sowohl das Signal des A/D-Wandlers 73 als auch das der Anzeige- und Registrieransteuerung 77 an.

Die Anzeige- und Registrieransteuerung 77 gibt bei jedem Korotkoff-Ton ein Signal, beginnend mit dem ersten, der dem systolischen Blutdruck entspricht, bis zum letzten, der dem diastolischen Blutdruck entspricht. Es sind zahlreiche Schaltungen bereits vorgeschlagen worden, mit denen dieses erreicht wird. Alle diese Schaltungen können für die vorliegende Erfindung verwendet werden, wobei selbstverständlich die Genauigkeit sowohl der unmittelbar angezeigten als auch der registrierten Blutdruckwerte wesentlich davon bestimmt wird, wie genau die Anzeige- und Registrieransteuerung 77 den ersten Korotkoff-Ton (systolischen Blutdruck) und das Verschwinden der Korotkoff-Töne (diastolischer Blutdruck) erkennt. Diese Schaltung ist jedoch nicht Gegenstand vorliegender Erfindung. Es soll daher nicht weiter auf diese Fragen eingegangen werden.

Die Verarbeitung der vom A/D-Wandler 73 und von der Anzeige- und Registrieransteuerung 77 gelieferten Signale zu einer digitalen Anzeige erfolgt durch die digitale Signalverarbeitung 78 und die eigentliche Anzeige 79 nach wohlbekannten Verfahren. In großen Zügen dargestellt, werden die vom A/D-Wandler kommenden, dem Druck entsprechenden digitalen Signale, bei denen die Anzeige- und Registrieransteuerung 77 ein Signal liefert, während des Ablassens des Blutdruckes abgespeichert. Aus dem Speicher können sie dann aufgerufen und über eine geeignete Umkodierungs- und Treiberschaltung auf einem 7-Segment-Display, das als Anzeige 79 dient, zur Darstellung gebracht werden.

Auch für die erfindungsgemäße Registrierung werden die dem systolischen und diastolischen Blutdruck entsprechenden digitalen Ausgangssignale des A/D-Wandlers 73 zunächst in einem Zwischenspeicher abgespeichert. Der Zwischenspeicher 80 enthält zusätzlich eine Rundungsschaltung. Da im allgemeinen die Anzahl der Druckelemente geringer ist als die Anzahl der digitalen Schritte, die in dem anzuzeigenden und zu registrierenden Druckbereich vom A/D-Wandler 73 geliefert werden, enthält der Zwischenspeicher 80 die Rundungsschaltung, die die Zahl der abzuspeichernden digitalen Zustände bereits entsprechend der Anzahl der Druckelemente 63 der Thermoleiste 55 reduziert. In einer konkreten Ausführungsform hat die Thermoleiste 55 zum Beispiel 256 Druckelemente zur Registrierung von Druckwerten zwischen 44 mm Hg und 300 mm Hg mit einer Auflösung von 1 mm Hg. Blutdruckwerte unter 44 mm Hg kommen praktisch nicht vor und müssen daher nicht registriert werden, auch wenn die Druckgraduierung 39 des Registrierblattes 31 aus graphischen Gründen bis 0 mm Hg geht.

Wenn nun der Blutdruck nach vorherigem Aufpumpen der Druckmanschette auf einen über dem systolischen Blutdruck liegenden Wert in bekannter Weise langsam abgelassen wird und der dem systolischen Blutdruck entsprechende Impuls der Anzeige- und Registrieransteuerung 77 beim Zwischenspeicher eintrifft, beginnt der Registriervorgang. Die, wie vor beschrieben, von der Rundungsschaltung gerundeten, jeweils einem der 256 Druckwerte entsprechenden Signale werden im Zwischenspeicher 80 jeweils abgespeichert, bis der Druckvorgang beendet ist. Gleichzeitig liegen die Signale an dem PROM-Umkodierer 81 an, der sie in eine geeignete Matrixansteuerung für die Zeilen und Spalten der Thermoleistenmatrix umwandelt. Wenn beim weiteren Ablassen des Luftdruckes die Anzeige- und Registrieransteuerung 77 den letzten Impuls gibt, weil die Korotkoff-Geräusche verschwinden (d. h. der diastolische Blutdruck erreicht ist), endet der Registriervorgang, d. h. es werden keine Impulse zur Ansteuerung von Heizwiderständen 63 der Thermoleiste 55 mehr an den PROM-Umkodierer 81 abgegeben.

Im vorliegenden Beispiel ist eine 16 × 16-Matrix für die Thermoleistenansteuerung besonders geeignet, die genau die 256 Heizwiderstände 63 der Thermoleiste 55 ansteuern kann. Um den notwendigen Heizstrom zu liefern, sind 16 Zeilentreiber 82 und 16 Spaltentreiber 83 zwischengeschaltet. Die genannten Zahlen sind besonders günstig, da die 16 Zustände für die 16 Spalten und ebenso die 16 Zustände für die 16 Zeilen gerade in jeweils 4 bit kodiert werden können. Je nach Ausführung der Elektronik können jedoch ohne großen Mehraufwand in einer jedem einschlägigen Fachmann bekannten Weise auch größere Zahlen von Druckelementen realisiert werden. Wie bereits zuvor erwähnt, ist die Zahl der Druckelemente bei Verwendung einer Thermoleiste weniger eine Frage des technischen und elektronischen Aufwandes, als eine Frage der graphischen Gestaltung der Registrierung. In diesem Sinne kann es durchaus sinnvoll sein, insbesondere für Verbrauchergeräte, die Anzahl der Druckelemente bewußt zu reduzieren, um ein besonders klares Bild zu erhalten.

Fig. 5 zeigt eine alternative Ausführungsform des Gerätes im Blockschaltbild, bei der ein Mikroprozessor zur Anwendung kommt. Der Mikroprozessor 84 übernimmt mit geeigneten Peripherieschaltungen, insbesondere einem Datenspeicher und einer Treiberstufe, die in dem Block 85 dargestellt sind, die Aufgaben der Blöcke 77, 78, 80, 81, 82 und 83 der zuvor dargestellten Ausführungsform. Die Mikroprozessor-Technologie ist heute allgemein bekannt, und es soll auf Einzelheiten daher hier nicht eingegangen werden.

Ein Vorteil der Realisierung mit Hilfe eines Mikroprozessors besteht darin, daß er geeignet ist, gleichzeitig eine Fehlererkennung für die vom Schmitt-Trigger 75 kommenden, den Korotkoff-Tönen entsprechen Signale durchzuführen. Eine derartige Fehlererkennung basiert auf der Fähigkeit des Mikroprozessors, logische Verknüpfungen zu vollziehen. Falsche Impulse können beispielsweise dadurch erkannt werden, daß der zeitliche Abstand zweier aufeinander folgender Signale vom mittleren Abstand der Korotkoff-Töne abweicht. Weitere Möglichkeiten der Ausnutzung eines Mikroprozessors sind bekannt und nicht Gegenstand der vorliegenden Erfindung.

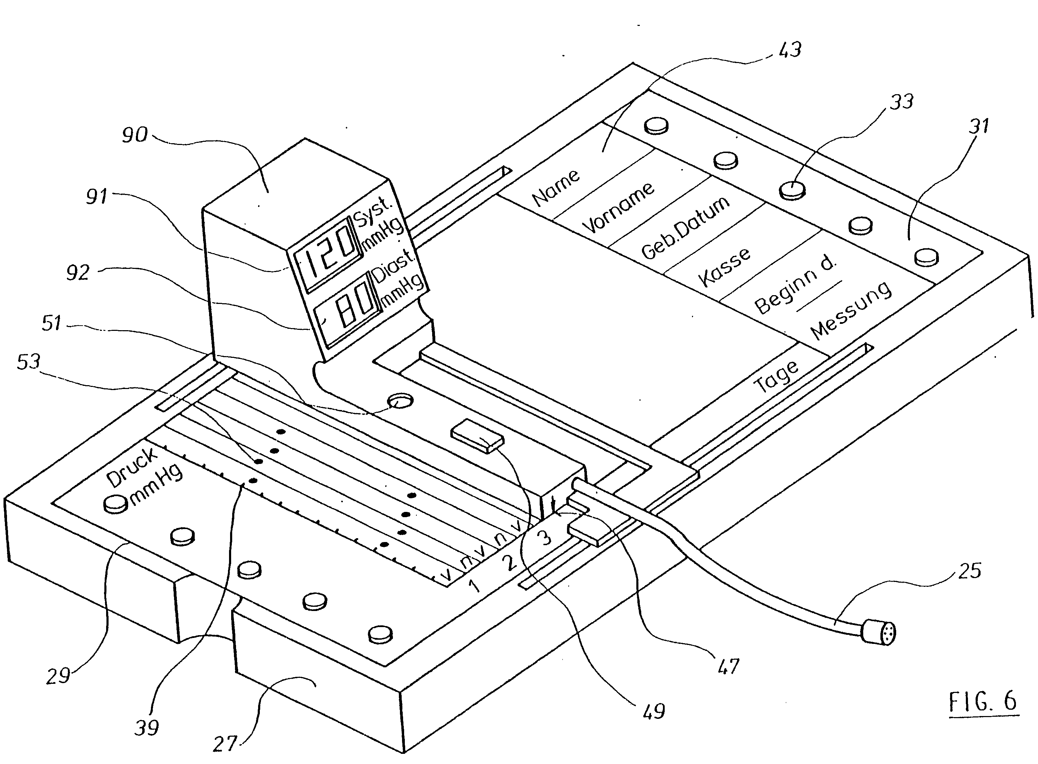

Fig. 6 zeigt eine alternative Ausführungsform einer erfindungsgemäßen Einrichtung, bei der die Registriervorrichtung gleichzeitig eine digitale Anzeige des Blutdruckes einschließt. Entsprechende Teile tragen die gleichen Bezugszeichen wie in Fig. 2. Der Schlitten beinhaltet gleichzeitig ein Gehäuse 90 mit zwei Anzeigefenstern 91 und 92 für den systolischen und diastolischen Blutdruck. Eine derartige Ausführungsform erlaubt eine besonders bequeme gleichzeitige Beobachtung der Anzeige und der registrierten Werte.

Die Bedienung der in den Fig. 2 und 6 dargestellten erfindungsgemäßen Registriervorrichtung ist sehr einfach. Zunächst wird das Registrierblatt 31 unter dem Schlitten 35 hindurchgeschoben, wobei es vorteilhaft ist, daß die Kanten 56 der Thermoleiste 55 abgerundet sind, wie in Fig. 3 zu erkennen ist. Außerdem ist zu diesem Zeitpunkt der Schalter 49 noch nicht betätigt, so daß die Thermoleiste 55 noch nicht oder nur sehr wenig von den Federn 57 belastet wird. Das Registrierblatt 31 wird über die Haltezapfen 33 gelegt und dadurch justiert. Anschließend kann eine Vielzahl von Blutdruckmessungen vorgenommen werden, wobei vor jeder neuen Blutdruckmessung der Schlitten in eine neue Position in horizontaler Richtung zu verschieben ist. Vor dem Registriervorgang wird der Schalter 49 betätigt, was durch die Betriebsanzeige 51 angezeigt wird. Dadurch wird die Druckplatte 59 (Fig. 3) nach unten bewegt und die Federn 57 belasten die Thermoleiste 55. Danach wird die Manschette aufgepumpt, der Blutdruck langsam abgelassen und der Meß- und Registriervorgang läuft in der zuvor beschriebenen Art und Weise ab. Nach Durchführung einer Reihe von Messungen, spätestens wenn das Registrierblatt 31 vollständig gefüllt ist, wird dieses dem Gerät entnommen und kann aufbewahrt und dem Arzt vorgelegt werden.

Bezugszeichenliste

  1 Druckmanschette
  3 Bügel
  5 Mikrofon
  7 Druckschlauch
  9 Mikrofonkabel
11 Anzeige- und Pumpeinheit
13 Pumpball
15 Druckablaßschraube
17 Anzeigekopf
19 Fenster von 17
21 Anschlußbuchse
23 Registriervorrichtung
25 Anschlußkabel von 23
27 Gehäuse von 23
29 Ausnehmung für 31
31 Registrierblatt
33 Haltezapfen

35 Schlitten
37 Führungsschlitze
39 Druckgraduierung
41 Zeitgraduierung
43 zusätzl. Informationsfelder
45 Sichtfenster
47 Justierpfeil
49 Schalter
51 Betriebsanzeige
53 Markierungspunkte
54 Auflage für 31
55 Thermoleiste
56 Kanten von 55
57 Federn
58 Substrat von 55
59 Druckplatte
61 Gehäuse von 35
63 Druckelement (Heizwiderstand)
65 Leitungen von 63
67 Leiterbahnen
72 Druckwandler
73 A/D-Wandler
74 Signalverstärkung und Filterung
75 Schmitt-Trigger
76 Leuchtdiode
77 Anzeige- und Registreier- ansteuerung
78 digitale Signalverarbeitung
79 Display
80 Zwischenspeicher
81 PROM-Umkodierer
82 Zeilentreiber
83 Spaltertreiber
84 Mikroprozessor
85 Datenspeicher und Treiberstufe
90 Gehäuse für digit. Anzeige
91, 92 dig. Blutdruckanzeigen

**Patentansprüche**

1. Vorrichtung zur Messung und Registrierung des Blutdruckes, umfassend
— eine aufblasbare Druckmanschette (1) zum Stauen des Blutes,
— eine Empfangseinrichtung (5) zum Empfangen der Korotkoff-Töne und Umwandeln derselben in elektrische Signale,
— eine Signalverarbeitunseinrichtung (74, 75) zum Aufbereiten der elektrischen Signale,
— eine Druckmeßeinrichtung (72, 73) zum Messen des Druckes in der Manschette und
— eine Registriereinrichtung (23) zur dauerhaften Aufzeichnung der Blutdruckwerte durch Markieren von mindestens zwei Druckwerten pro Messung mit Hilfe einer Markierungseinrichtung auf einem Registrierblatt (31) mit einer druckgraduierten Skala (39), wobei die beiden Druckwerte näherungsweise dem ersten und letzten empfangenen Korotkoff-Ton, also dem systolischen und diastolischen Blutdruck entsprechen, dadurch gekennzeichnet, daß
— das Registrierblatt (31) Bereiche für eine Mehrzahl von zu verschiedenen Zeitpunkten gewonnenen Meßergebnissen aufweist,
— die Markierungseinrichtung einen relativ zum Registrierblatte (31), insbesondere manuell, beweglichen und in verschiedene Positionen einstellbaren Schlitten (35) aufweist, wobei jede Position einem verschiedenen Meßzeitpunkt entspricht,
— an dem Schlitten (35) eine Mehrzahl, insbesondere punktförmige Markierungen (53) erzeugende, Druckelemente (63) in einer relativ zum Schlitten (35) festen Position angebracht sind, wobei jedes Element (63) einem bestimmten Druckbereich der Druckmeßeinrichtung entspricht,
— die Signalverarbeitungseinrichtung (74, 75) digitalisierende Elemente zur Erzeugung definierter Impulse aus den Korotkoff-Geräuschen aufweist und
— eine Ansteuereinrichtung (77, 80, 81, 82, 83) vorgesehen ist, durch die die Druckelemente (63) angesteuert werden, wenn in dem dem jeweiligen Element (63) entsprechenden Druckbereich ein mindestens dem ersten und dem letzten empfangenen Korotkoff-Ton entsprechender definierter Impuls von der Signalverarbeitungseinrichtung (74, 75) empfangen wird.

2. Vorrichtung nach Anspruch 1, dadurch gekennzeichnet, daß die Druckelemente (63) bewegungslos ausgeführt sind, wobei eine Andruckeinrichtung (57, 59) vorgesehen ist, durch die die bewegungslosen Druckelemente (63) und das Registrierblatt (31) in engen Kontakt gebracht werden.

3. Vorrichtung nach Anspruch 2, dadurch gekennzeichnet, daß die Andruckeinrichtung (57, 59) eine im Schlitten (35) untergebrachte, das Substrat (58) der Druckelemente (63) belastende Federeinrichtung (57) ist.

4. Vorrichtung nach einem der Ansprüche 2 oder 3, dadurch gekennzeichnet, daß die Druckelemente (63) thermisch drückende Elemente sind.

5. Vorrichtung nach einem der Ansprüche 1 bis 4, dadurch gekennzeichnet, daß die Ansteuereinrichtung (77, 80, 81, 82, 83) mittels einer Speicherschaltung so ausgebildet ist, daß nur diejenigen Impulse zur Ansteuerung eines Druckelementes (63) führen, die dem dem systolischen Blutdruckwert und dem dem diastolischen Blutdruckwert nächstliegenden Korotkoff-Ton entsprechen.

6. Vorrichtung nach einem der Ansprüche 1 bis 5, dadurch gekennzeichnet, daß das Registrieblatt (31) ein rechtwinkliges Koordinatensystem mit der Abszisse als Zeitachse (41) und der Ordinate als Druckachse (39) bildet, wobei die Bereiche aneinander angrenzen, spaltenförmig sind und parallel zur Ordinate verlaufen, so daß die punktförmigen Markierungen (53), die einem oder mehreren bestimmten charakteristischen Blutdruckwerten, insbesondere dem systolischen oder dem diastolischen Blutdruckwert, zuzuordnen sind, einen oder mehrere charakteristische zeitabhängige Kurvenzüge ergeben.

7. Vorrichtung nach einem der Ansprüche 1 bis 6, dadurch gekennzeichnet, daß die Druckelemente (63) an dem Schlitten (35) in gleichmäßigen Abständen angeordnet sind und äquidistanten Druckbereichen entsprechen, so daß sich eine lineare Blutdruckanzeige ergibt.

8. Vorrichtung nach einem der Ansprüche 1 bis 7, dadurch gekennzeichnet, daß zusätzlich zur Registriereinrichtung eine digitale Anzeigeeinrichtung zur Anzeige der Blutdruckwerte vorhanden ist.

9. Vorrichtung nach einem der Ansprüche 1 bis 8, dadurch gekennzeichnet, daß die Registriereinrichtung (23) separat von einem die Signalverarbeitungseinrichtung, die Druckmeßeinrichtung und gegebenenfalls die zusätzliche Anzeige einschließenden Blutdruckmeßgerat (1, 11) in einem besonderen Gehäuse untergebracht und wahlweise an das Blutdruckmeßgerät anschließbar ist.

10. Vorrichtung nach einem der Ansprüche 1 bis 9, dadurch gekennzeichnet, daß die Ansteuereinrichtung einen Mikroprozessor (84) einschließt, der gleichzeitig zur Fehlererkennung der von der Empfangseinrichtung (5) kommenden elektrischen Signale verwendet wird.

## Claims

1. Device for the measurement and recordal of the blood pressure, comprising
— an inflatable pressure cuff (1) for the damming of the blood,
— a receiving device (5) for the reception of the Korotkoff sounds and conversion thereof into electrical signals,
— a signal processing device (74, 75) for the processing of the electrical signals,
— a pressure measurement device (72, 73) for the measurement of the pressure in the cuff and
— a recordal device (23) for the permanent recordal of the blood pressure values by marking of at least two pressure values per measurement with the help of a marking device on a recordal sheet (31) with a pressure-graduated scale (39), whereby the two pressure values correspond approximately to the first and the last received Korotkoff sound, thus to the systolic and the diastolic blood pressure,
characterised in that
— the recordal sheet (31) has a region for a plurality of measurement results obtained at different points of time,
— the marking device has a slider (35) movable relative to the recordal sheet (31), especially manually, and adjustable in various positions, whereby each position corresponds to a different measurement time point,
— on the slider (35) are provided a plurality of pressure elements (63), especially producing punctate markings (53), in a fixed position relative to the slider (35), whereby each element (63) corresponds to a definite pressure region of the pressure measurement device.
— the signal processing device (74, 75) has digitalising elements for the production of definite impulses from the Korotkoff noises and

— a directing device (77, 80, 81, 82, 83) is provided by means of which the pressure elements (63) are directed when, in the pressure region corresponding to the particular element (63), there is received a definite impulse from the signal processing device (74, 75) corresponding at least to the first and last received Korotkoff sound.

2. Device according to claim 1, characterised in that the pressure elements (63) are made non-moving, whereby a pressing-on device (57, 59) is provided by means of which the non-moving pressure element (63) and the recording sheet (31) are brought into close contact.

3. Device according to claim 2, characterised in that the pressing on device (57, 59) is a spring device (57) housed in the slider (35), loading the substrate (58) of the pressure element (63).

4. Device according to one of claims 2 or 3, characterised in that the pressure elements (63) are thermal pressing elements.

5. Device according to one of claims 1 to 4, characterised in that the directing device (77, 80, 81, 82, 83) is so constructed by means cf a storage circuit that only those impulses lead to a directing of a pressure element (63) which correspond to the Korotkoff sound lying closest to the systolic blood pressure value and to the diastolic blood pressure value.

6. Device according to one of claims 1 to 5, characterised in that the recordal sheet (31) forms a rectangular coordinate system with the abscissa as time axis (41) and the ordinate as pressure axis (39), whereby the regions bound one another, are column-shaped and run parallel to the ordinate so that the punctate markings (53), which are to be associated with one or more definite characteristic blood pressure values, especially with the systolic or the diastolic blood pressure value, give one or more characteristic, time-dependent curves.

7. Device according to one of claims 1 to 6. characterised in that the pressure elements (63) are arranged on the slider (35) at uniform distances and correspond to equidistant pressure ranges so that there is given a linear blood pressure indication.

8. Device according to one of claims 1 to 7, characterised in that, in addition to the recordal device, there is present a digital indicating device for the indication of the blood pressure values.

9. Device according to one of claims 1 to 8, characterised in that the recordal device (23) is housed separately from a blood measurement apparatus (1, 11), including the signal processing device, the pressure measurement device and possibly the additional indicator, in a separate housing and can be connected, as desired, to the blood pressure measurement apparatus.

10. Device according to one of claims 1 to 9, characterised in that the directing device includes a microprocessor (84) which is used simultaneously for the recognition of errors of the electrical signals coming from the receiving device (5).

## Revendications

1. Dispositif pour mesurer et enregistrer la pression artérielle comportant :
— un manchon de compression pneumatique (1) pour le refoulement du sang,
— un dispositif récepteur (5) pour la réception des bruits de Korotkoff et pour leur conversion en signaux électriques,
— un dispositif pour le traitement de signaux (74, 75) pour le traitement des signaux électriques,
— un dispositif pour la mesure de pressions (72, 73) pour mesurer la pression dans le manchon, et
— un enregistreur pour l'enregistrement continu des valeurs de la pression artérielle par le marquage d'au moins deux valeurs de cette pression par mesure au moyen d'un dispositif de marquage sur une feuille d'enregistrement (31) pourvue d'une échelle (39) graduée en pressions, les deux valeurs de pression correspondant approximativement au premier et au dernier bruits de Korotkoff reçus, c'est-à-dire aux pressions artérielles systolique et diastolique, caractérisé en ce que
— la feuille d'enregistrement (31) présente des plages pour une pluralité de résultats de mesure obtenus à des moments différents,
— le dispositif de marquage est pourvu d'un coulisseau (35) mobile, en particulier manuellement, par rapport à la feuille d'enregistrement (31) et pouvant être placé en des positions différentes, chaque position correspondant à un moment de mesure différent,
— sur le coulisseau (35) sont montés en position fixe par rapport au coulisseau (35), une pluralité d'éléments imprimants (63), en particulier du type imprimant des marques en forme de point, chaque élément (63) correspondant à un certain domaine de pressions du dispositif pour la mesure de pressions,
— le dispositif pour le traitement de signaux (74, 75) présente des éléments de digitalisation pour la création d'impulsions définies à partir des bruits de Korotkoff et
— il est prévu un dispositif de sélection (77, 80, 81, 82, 83) assurant la sélection des éléments imprimants (63), au moment où dans le domaine de pression correspondant à un élément (63) déterminé, une impulsion provenant du dispositif pour le traitement de signaux (74, 75) est reçue dont la définition correspond au moins au premier et au dernier bruit de Korotkoff reçu.

2. Dispositif selon la revendication 1, caractérisé en ce que les éléments imprimants (63) sont réalisés immobiles, un dispositif d'appui (57, 59) étant prévu qui met les éléments imprimants (63) immobiles et la feuille d'enregistrement (31) en contact intime.

3. Dispositif selon la revendication 2, caractérisé en ce que le dispositif d'appui (57, 59) consiste en un dispositif à ressort (57) placé dans le coulisseau (35) et mettant le support (58) des éléments imprimants (63) sous pression.

4. Dispositif selon l'une des revendications 2 ou 3, caractérisé en ce que les éléments imprimants (63) sont du type à impression par effet thermique.

5. Dispositif selon l'une quelconque des revendications 1 à 4, caractérisé en ce que le dispositif de sélection (77, 80, 81, 82, 83) est réalisé au moyen d'un circuit à mémoire de telle sorte que seules les impulsions correspondant à la pression artérielle systolique et au bruit de Korotkoff le plus rapproché de la pression artérielle diastolique, conduisent à la sélection d'un élément imprimant (63).

6. Dispositif selon l'une quelconque des revendications 1 à 5, caractérisé en ce que la feuille d'enregistrement (31) forme un système de coordonnées orthogonales dans lequel l'abscisse comporte les temps (41) et l'ordonnée les pressions (39), les plages étant contiguës, en forme de colonnes et parallèles à l'ordonnée, de sorte que les marques (53) en forme de point représentant une ou plusieurs valeurs déterminées caractéristiques de la pression artérielle, en particulier de la pression artérielle systolique ou diastolique, forment une ou plusieurs courbes caractéristiques en fonction du temps.

7. Dispositif selon l'une quelconque des revendications 1 à 6, caractérisé en ce que les éléments imprimants (63) sont disposés à des distances égales sur le coulisseau (35) et correspondent à des plages de pression équidistantes, de sorte qu'il en résulte une indication linéaire de la pression artérielle.

8. Dispositif selon l'une quelconque des revendications 1 à 7, caractérisé en ce que complémentairement au dispositif d'enregistrement il est prévu un dispositif pour l'affichage des valeurs de la pression artérielle.

9. Dispositif selon l'une quelconque des revendications 1 à 8, caractérisé en ce que le dispositif d'enregistrement (23) est logé dans un boîtier spécial séparément de l'appareil pour la mesure de la pression artérielle (1, 11) comprenant le dispositif pour le traitement de signaux, le dispositif pour la mesure de pressions et éventuellement l'afficheur complémentaire, et peut être relié à cet appareil pour la mesure de la pression artérielle en cas de besoin.

10. Dispositif selon l'une quelconque des revendications 1 à 9, caractérisé en ce que le dispositif de sélection comporte un microprocesseur (84) mis en œuvre simultanément pour la détection d'erreurs dans les signaux électriques provenant du dispositif récepteur (5).

FIG.1

Systole 123

Diastole 79

0 072 823

FIG. 2

Name  Vorname  Geb. Datum  Kasse  Beginn d  Messung

Tage

Druck mmHg

FIG. 3

3

FIG. 4

FIG. 5

FIG. 6